# EUROPEAN PATENT APPLICATION

(11) **EP 4 582 450 A1**
(43) Date of publication of application: **09.07.2025**
(21) Application number: 23859277.8
(22) Date of filing: 28.08.2023
(51) Int. Cl.: C07K 16/28, C12N 15/13, C12N 15/85, C12N 5/10, A61K 39/395, A61P 35/00

(54) **ANTI-PVRIG ANTIBODY AND APPLICATION THEREOF**

(30) Priority: 31.08.2022 CN 202211052389
(71) Applicant: CSPC Megalith Biopharmaceutical Co., Ltd., Shijiazhuang, Hebei 050025 (CN)
(72) Inventor: DING, Huandi, Shijiazhuang, Hebei 050025 (CN); HUI, Xiwu, Shijiazhuang, Hebei 050025 (CN); YAO, Bing, Shijiazhuang, Hebei 050025 (CN); DAN, Mo, Shijiazhuang, Hebei 050025 (CN); WANG, Ruihuan, Shijiazhuang, Hebei 050025 (CN); FENG, Jing, Shijiazhuang, Hebei 050025 (CN); LIU, Jieru, Shijiazhuang, Hebei 050025 (CN); WANG, Mingxiao, Shijiazhuang, Hebei 050025 (CN)
(74) Representative: Lavoix
(86) International application number: PCT/CN2023/115147
(87) International publication number: WO 2024/046245

(57) **Abstract**

Disclosed are an anti-human PVRIG antibody or an antigen-binding fragment thereof, as well as an application thereof in the preparation of a medicament for treating diseases, such as cancer, which are related to the abnormal expression of PVRIG.

## Description

### TECHNICAL FIELD

The present application belongs to the field of biomedicine, and particularly relates to an anti-human PVRIG antibody or an antigen-binding fragment thereof and use thereof.

### BACKGROUND

Human PVRIG (poliovirus receptor-related immunoglobin domain-containing protein), also known as CD112 receptor (CD112R), is a 34kda protein in the poliovirus receptor-like protein (PVR) family. This gene encodes a transmembrane protein consisting of: an extracellular IgV domain, a transmembrane domain, and a long intracellular domain. PVRIG (CD112R) is mainly expressed on T cells and NK cells, and plays a regulatory role in immune function. When PVRIG binds to its ligand PVRL2 (CD112, Nectin-2) on antigen presenting cells, it inhibits immune activation. In addition, PVRIG can also exert an immunosuppressive effect by competitively binding to CD226 ligands and blocking the stimulation signals transmitted by it. This is similar to the immunosuppression mechanism of its family member TIGIT, and the two may have a synergistic effect in immunosuppression. Clinically, PVRL2 has been found to be overexpressed in multiple tumor types including breast cancer and ovarian cancer, indicating that its interaction with PVRIG may be a major mechanism of immunosuppression in these diseases. Therefore, therapeutic strategies of targeting PVRIG have the potential to be applied clinically as single-drug, dual-antibody, or combination therapy. Currently, the most advanced ones are COM-701 from Compugen and SRF813 from Surface Oncology, and they are in the clinical research stage and no products are on the market yet.

In order to provide patients with more diversified treatment options, the present application obtains antibodies with higher affinity for recognizing PVRIG on the cell surface, and compared with SRF813 (patent US20210253699A1), the antibodies of the present application can block the binding of PVRIG to CD112 at the cellular level to a greater extent. In addition, in vivo animal experimental data confirm that the antibodies obtained by the present application can effectively inhibit the excessive proliferation of tumor cells.

### SUMMARY

In one aspect, the present application relates to an anti-human PVRIG antibody or an antigen-binding fragment thereof, comprising:
(1) HCDR1 comprising the amino acid sequence of SEQ ID NO: 1, HCDR2 comprising the amino acid sequence of SEQ ID NO: 2, and HCDR3 comprising the amino acid sequence of SEQ ID NO: 3; and/or LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6; or
(2) HCDR1 comprising the amino acid sequence of SEQ ID NO: 17, HCDR2 comprising the amino acid sequence of SEQ ID NO: 18 or SEQ ID NO: 19 or SEQ ID NO: 20, and HCDR3 comprising the amino acid sequence of SEQ ID NO: 21; and/or LCDR1 comprising the amino acid sequence of SEQ ID NO: 22 or SEQ ID NO: 23, LCDR2 comprising the amino acid sequence of SEQ ID NO: 24 or SEQ ID NO: 25, and LCDR3 comprising the amino acid sequence of SEQ ID NO: 26; or
(3) HCDR1 comprising the amino acid sequence of SEQ ID NO: 36, HCDR2 comprising the amino acid sequence of SEQ ID NO: 37, and HCDR3 comprising the amino acid sequence of SEQ ID NO: 38; and/or LCDR1 comprising the amino acid sequence of SEQ ID NO: 39, LCDR2 comprising the amino acid sequence of SEQ ID NO: 40, and LCDR3 comprising the amino acid sequence of SEQ ID NO: 41.

In a particular embodiment of the present application, provided is an anti-human PVRIG antibody or an antigen-binding fragment thereof, comprising:
(1) HCDR1 comprising the amino acid sequence of SEQ ID NO: 1, HCDR2 comprising the amino acid sequence of SEQ ID NO: 2, and HCDR3 comprising the amino acid sequence of SEQ ID NO: 3; and LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6; or
(2) HCDR1 comprising the amino acid sequence of SEQ ID NO: 17, HCDR2 comprising the amino acid sequence of SEQ ID NO: 20, and HCDR3 comprising the amino acid sequence of SEQ ID NO: 21; and LCDR1 comprising the amino acid sequence of SEQ ID NO: 23, LCDR2 comprising the amino acid sequence of SEQ ID NO: 25, and LCDR3 comprising the amino acid sequence of SEQ ID NO: 26; or
(3) HCDR1 comprising the amino acid sequence of SEQ ID NO: 36, HCDR2 comprising the amino acid sequence of SEQ ID NO: 37, and HCDR3 comprising the amino acid sequence of SEQ ID NO: 38; and LCDR1 comprising the amino acid sequence of SEQ ID NO: 39, LCDR2 comprising the amino acid sequence of SEQ ID NO: 40, and LCDR3 comprising the amino acid sequence of SEQ ID NO: 41.

In one embodiment of the present application, provided is an anti-human PVRIG antibody or an antigen-binding fragment thereof, comprising a heavy chain variable region and/or a light chain variable region, wherein:
(1) the heavy chain variable region comprises an amino acid sequence selected from SEQ ID NO: 7, SEQ ID NO: 9, SEQ ID NO: 10, SEQ ID NO: 11 or SEQ ID NO: 12, and/or the light chain variable region comprises an amino acid sequence selected from SEQ ID NO: 8, SEQ ID NO: 13, SEQ ID NO: 14, SEQ ID NO: 15 or SEQ ID NO: 16; or
(2) the heavy chain variable region comprises an amino acid sequence selected from SEQ ID NO: 27, SEQ ID NO: 29, SEQ ID NO: 30, SEQ ID NO: 31 or SEQ ID NO: 32, and/or the light chain variable region comprises an amino acid sequence selected from SEQ ID NO: 8, SEQ ID NO: 33, SEQ ID NO: 34 or SEQ ID NO: 35; or
(3) the heavy chain variable region comprises an amino acid sequence selected from SEQ ID NO: 42, SEQ ID NO: 44, SEQ ID NO: 45, SEQ ID NO: 46 or SEQ ID NO: 47, and/or the light chain variable region comprises an amino acid sequence selected from SEQ ID NO: 43, SEQ ID NO: 48, SEQ ID NO: 49 or SEQ ID NO: 50.

In one embodiment of the present application, provided is an anti-human PVRIG antibody or an antigen-binding fragment thereof, comprising a heavy chain variable region and/or a light chain variable region, wherein:
(1) the heavy chain variable region comprises the amino acid sequence of SEQ ID NO: 47, and/or the light chain variable region comprises the amino acid sequence of SEQ ID NO: 50; or
(2) the heavy chain variable region comprises the amino acid sequence of SEQ ID NO: 32, and/or the light chain variable region comprises the amino acid sequence of SEQ ID NO: 35; or
(3) the heavy chain variable region comprises the amino acid sequence of SEQ ID NO: 10, and/or the light chain variable region comprises the amino acid sequence of SEQ ID NO: 14.

In one embodiment of the present application, provided is an anti-human PVRIG antibody or an antigen-binding fragment thereof, comprising a heavy chain variable region and/or a light chain variable region, wherein:
(1) the heavy chain variable region comprises the amino acid sequence of SEQ ID NO: 47, and the light chain variable region comprises the amino acid sequence of SEQ ID NO: 50; or
(2) the heavy chain variable region comprises the amino acid sequence of SEQ ID NO: 32, and the light chain variable region comprises the amino acid sequence of SEQ ID NO: 35; or
(3) the heavy chain variable region comprises the amino acid sequence of SEQ ID NO: 10, and the light chain variable region comprises the amino acid sequence of SEQ ID NO: 14.

In one embodiment of the present application, provided is an anti-human PVRIG antibody or an antigen-binding fragment thereof, comprising a heavy chain variable region and/or a light chain variable region, wherein:
(1) the heavy chain variable region comprises a sequence selected from the amino acid sequences respectively having 80%, 85%, 90%, 95% or 99% homology with SEQ ID NO: 7, SEQ ID NO: 9, SEQ ID NO: 10, SEQ ID NO: 11 or SEQ ID NO: 12, and/or the light chain variable region comprises a sequence selected from the amino acid sequences respectively having 80%, 85%, 90%, 95% or 99% homology with SEQ ID NO: 8, SEQ ID NO: 13, SEQ ID NO: 14, SEQ ID NO: 15 or SEQ ID NO: 16; or
(2) the heavy chain variable region comprises a sequence selected from the amino acid sequences respectively having 80%, 85%, 90%, 95% or 99% homology with SEQ ID NO: 27, SEQ ID NO: 29, SEQ ID NO: 30, SEQ ID NO: 31 or SEQ ID NO: 32, and/or the light chain variable region comprises a sequence selected from the amino acid sequences respectively having 80%, 85%, 90%, 95% or 99% homology with SEQ ID NO: 8, SEQ ID NO: 33, SEQ ID NO: 34 or SEQ ID NO: 35; or
(3) the heavy chain variable region comprises a sequence selected from the amino acid sequences respectively having 80%, 85%, 90%, 95% or 99% homology with SEQ ID NO: 42, SEQ ID NO: 44, SEQ ID NO: 45, SEQ ID NO: 46 or SEQ ID NO: 47, and/or the light chain variable region comprises a sequence selected from the amino acid sequences respectively having 80%, 85%, 90%, 95% or 99% homology with SEQ ID NO: 43, SEQ ID NO: 48, SEQ ID NO: 49 or SEQ ID NO: 50.

In one embodiment of the present application, provided is an anti-human PVRIG antibody or an antigen-binding fragment thereof, comprising a heavy chain variable region and/or a light chain variable region, wherein:
(1) the heavy chain variable region comprises a sequence selected from the amino acid sequences respectively having 80%, 85%, 90%, 95% or 99% homology with the amino acid sequence of SEQ ID NO: 47, and/or the light chain variable region comprises a sequence selected from the amino acid sequences respectively having 80%, 85%, 90%, 95% or 99% homology with the amino acid sequence of SEQ ID NO: 50; or
(2) the heavy chain variable region comprises a sequence selected from the amino acid sequences respectively having 80%, 85%, 90%, 95% or 99% homology with the amino acid sequence of SEQ ID NO: 32, and/or the light chain variable region comprises a sequence selected from the amino acid sequences respectively having 80%, 85%, 90%, 95% or 99% homology with the amino acid sequence of SEQ ID NO: 35; or
(3) the heavy chain variable region comprises a sequence selected from the amino acid sequences respectively having 80%, 85%, 90%, 95% or 99% homology with the amino acid sequence of SEQ ID NO: 10, and/or the light chain variable region comprises a sequence selected from the amino acid sequences respectively having 80%, 85%, 90%, 95% or 99% homology with the amino acid sequence of SEQ ID NO: 14.

In one embodiment of the present application, provided is an anti-human PVRIG antibody or an antigen-binding fragment thereof, which comprising a heavy chain variable region and a light chain variable region, wherein:
(1) the heavy chain variable region comprises a sequence selected from the amino acid sequences respectively having 80%, 85%, 90%, 95% or 99% homology with the amino acid sequence of SEQ ID NO: 47, and the light chain variable region comprises a sequence selected from the amino acid sequences respectively having 80%, 85%, 90%, 95% or 99% homology with the amino acid sequence of SEQ ID NO: 50; or
(2) the heavy chain variable region comprises a sequence selected from the amino acid sequences respectively having 80%, 85%, 90%, 95% or 99% homology with the amino acid sequence of SEQ ID NO: 32, and the light chain variable region comprises a sequence selected from the amino acid sequences respectively having 80%, 85%, 90%, 95% or 99% homology with the amino acid sequence of SEQ ID NO: 35; or
(3) the heavy chain variable region comprises a sequence selected from the amino acid sequences respectively having 80%, 85%, 90%, 95% or 99% homology with the amino acid sequence of SEQ ID NO: 10, and the light chain variable region comprises a sequence selected from the amino acid sequences respectively having 80%, 85%, 90%, 95% or 99% homology with the amino acid sequence of SEQ ID NO: 14.

In one embodiment of the present application, the anti-human PVRIG antibody or an antigen-binding fragment thereof is preferably a monoclonal antibody.

In one embodiment of the present application, the anti-human PVRIG antibody or an antigen-binding fragment thereof is a humanized antibody or human antibody, or a portion thereof.

In one embodiment of the present application, the anti-human PVRIG antibody or an antigen-binding fragment thereof further comprises an Fc region.

In one embodiment of the present application, the anti-human PVRIG antibody or an antigen-binding fragment thereof is an IgG1 or IgG4 antibody, or a portion thereof.

In one embodiment of the present application, the anti-human PVRIG antibody or an antigen-binding fragment thereof further comprises an Fc region variant.

In one embodiment of the present application, the Fc variant comprises an amino acid sequence different from a "native" or "wild-type" Fc region sequence by having at least one amino acid mutation as compared to the native Fc region sequence. Preferably, the Fc variant has at least one amino acid substitution as compared to the native Fc region sequence or to the Fc region sequence of a parent polypeptide, for example, from about 1-10 amino acid substitutions in the native Fc region sequence or in the Fc region sequence of a parent polypeptide, and preferably from about 1-5 amino acid substitutions. Preferably, the Fc region variant herein will have at least about 80% homology with a native Fc region sequence and/or with the Fc region sequence of a parent polypeptide, and most preferably at least about 90% homology therewith, and more preferably at least about 95% homology therewith.

In one embodiment of the application, the Fc region variant is an IgG1 Fc variant sequence.

In one embodiment of the present application, the mutation in the IgG1 Fc variant occurs at serine at position 239, alanine at position 330, or isoleucine at position 332.

In one embodiment of the present application, the mutations in the IgG1 Fc variant occur at serine at position 239, alanine at position 330, and isoleucine at position 332.

In one embodiment of the present application, the mutations in the IgG1 Fc variant comprise S239D, A330L and I332E triple mutations.

The Fc variants of the present application are defined according to the amino acid modifications that constitute them. Thus, for example, P329G is an Fc variant having a substitution of glycine for proline at position 329 relative to the parent Fc polypeptide, wherein numbering is according to the EU index. The identity of the wild-type amino acid may not be specified, in this case the variant described above is referred to as P329G. For all positions in the Fc region discussed in the present application, the numbering is according to the EU index. The EU index, or the EU index or the EU numbering scheme as in Kabat refers to the EU numbering for antibodies (Edelman et al., Proc Natl Acad Sci USA 63 (1969) 78-85, which is incorporated herein by reference in its entirety).

In one embodiment of the present application, the antigen-binding fragment of the anti-human PVRIG antibody is selected from the group consisting of: Fab, Fab', F(ab')2, Fd, Fv, scFv, dAb or CDR.

Unless otherwise indicated, numbering of the variable region amino acids herein is determined according to the Kabat numbering convention.

In another aspect of the present application, provided is an isolated nucleic acid molecule encoding the above anti-human PVRIG antibody or an antigen-binding fragment thereof.

In the third aspect of the present application, provided is an expression vector comprising the above nucleic acid molecule.

In the fourth aspect of the present application, provided is a host cell comprising the above expression vector.

In the fifth aspect of the present application, provided is a method for preparing the above anti-human PVRIG antibody or an antigen-binding fragment thereof, which includes culturing the above host cell, thereby expressing the nucleic acid molecule.

In one embodiment of the present application, the method for preparing the above anti-human PVRIG antibody or an antigen-binding fragment thereof further includes the step of recovering the anti-human PVRIG antibody or an antigen-binding fragment thereof from the host cell culture.

In the sixth aspect of the present application, provided is an antibody-drug conjugate comprising the above anti-human PVRIG antibody or an antigen-binding fragment thereof linked to a therapeutic agent.

In the seventh aspect of the present application, provided is a bispecific molecule comprising the above anti-PVRIG antibody or an antigen-binding fragment thereof linked to a peptide targeting another antigen epitope or another antigen.

In the eighth aspect of the present application, provided is a composition comprising the above anti-human PVRIG antibody or an antigen-binding fragment thereof, or the above antibody-drug conjugate, or the above bispecific molecule; optionally, the composition further comprises a pharmaceutically acceptable carrier.

In the ninth aspect of the present application, provided is use of the above anti-human PVRIG antibody or an antigen-binding fragment thereof, or the above antibody-drug conjugate, or the above bispecific molecule, or the above composition in the preparation of a medicament for regulating immune response in a subject.

In the tenth aspect of the present application, provided is a method for regulating immune response in a subject, comprising administering to the subject a therapeutically effective amount of the above anti-human PVRIG antibody or an antigen-binding fragment thereof, or the above antibody-drug conjugate, or the above bispecific molecule, or the above composition.

In the eleventh aspect of the present application, provided is the above anti-human PVRIG antibody or an antigen-binding fragment thereof, or the above antibody-drug conjugate, or the above bispecific molecule, or the above composition, for use in regulating immune response in a subject.

In the twelfth aspect of the present application, provided is use of the above anti-human PVRIG antibody or an antigen-binding fragment thereof, or the above antibody-drug conjugate, or the above bispecific molecule, or the above composition in the preparation of a medicament for inhibiting the growth of tumor cells in a subject.

In the thirteenth aspect of the present application, provided is a method for inhibiting tumor cell growth in a subject, comprising administering to the subject a therapeutically effective amount of the above anti-human PVRIG antibody or an antigen-binding fragment thereof, or the above antibody-drug conjugate, or the above bispecific molecule, or the above composition.

In the fourteenth aspect of the present application, provided is the above anti-human PVRIG antibody or an antigen-binding fragment thereof, or the above antibody-drug conjugate, or the above bispecific molecule, or the above composition, for use in inhibiting tumor cell growth in a subject.

In a particular embodiment of the present application, the tumor cell is a tumor cell overexpressing PVRIG or PVRL2. In a particular embodiment of the present application, the tumor cells are selected from tumor cells of: bone cancer, pancreatic cancer, skin cancer, head or neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, cancer of the endocrine system, thyroid cancer, parathyroid cancer, adrenal cancer, soft tissue sarcoma, urethral cancer, penile cancer, chronic or acute leukemia, acute myeloid leukemia, chronic myeloid leukemia, acute lymphocytic leukemia, chronic lymphocytic leukemia, childhood solid tumor, lymphocytic lymphoma, bladder cancer, kidney or ureter cancer, renal pelvis cancer, central nervous system (CNS) neoplasms, primary CNS lymphomas, tumor angiogenesis, spinal cord axis tumor, brain stem gliomas, pituitary adenomas, Kaposi's sarcoma, epidermoid carcinomas, squamous cell carcinomas, T-cell lymphomas, and any combination of the above cancers.

In the fifteenth aspect of the present application, provided is use of the above anti-human PVRIG antibody or an antigen-binding fragment thereof, or the above antibody-drug conjugate, or the above bispecific molecule, or the above composition in the preparation of a medicament for treating a hyperproliferative disease.

In the sixteenth aspect of the present application, provided is a method for treating a hyperproliferative disease, comprising administering to a subject a therapeutically effective amount of the above anti-human PVRIG antibody or an antigen-binding fragment thereof, or the above antibody-drug conjugate, or the above bispecific molecule, or the above combination.

In the seventeenth aspect of the present application, provided is a therapeutically effective amount of the above anti-human PVRIG antibody or an antigen-binding fragment thereof, or the above antibody-drug conjugate, or the above bispecific molecule, or the above composition, for use in treating a hyperproliferative disease.

In a particular embodiment of the present application, the hyperproliferative disease is a cancer, preferably a cancer that overexpresses PVRL2.

In a particular embodiment of the application, the cancer is selected from the group consisting of: bone cancer, pancreatic cancer, skin cancer, head or neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, cancer of the endocrine system, thyroid cancer, parathyroid cancer, adrenal cancer, soft tissue sarcoma, urethral cancer, penile cancer, chronic or acute leukemia, acute myeloid leukemia, chronic myeloid leukemia, acute lymphocytic leukemia, chronic lymphocytic leukemia, childhood solid tumor, lymphocytic lymphoma, bladder cancer, kidney or ureter cancer, renal pelvis cancer, central nervous system (CNS) neoplasms, primary CNS lymphomas, tumor angiogenesis, spinal cord axis tumor, brain stem gliomas, pituitary adenomas, Kaposi's sarcoma, epidermoid carcinomas, squamous cell carcinomas, T-cell lymphomas, and any combination of the above cancers.

In the eighteenth aspect of the present application, provided is use of the above anti-human PVRIG antibody or an antigen-binding fragment thereof, or the above antibody-drug conjugate, or the above bispecific molecule, or the above composition in the preparation of a medicament for blocking the binding of PVRIG protein to CD112 protein.

In the nineteenth aspect of the present application, provided is a method for blocking the binding of PVRIG protein to CD112 protein, comprising administering to a subject a therapeutically effective amount of the above anti-human PVRIG antibody or an antigen-binding fragment thereof, or the above antibody-drug conjugate, or the above bispecific molecule, or the above composition.

In the twentieth aspect of the present application, provided is the above anti-human PVRIG antibody or an antigen-binding fragment thereof, or the above antibody-drug conjugate, or the above bispecific molecule, or the above composition, for use in blocking the binding of PVRIG protein to CD112 protein.

### Definitions

The term "antibody" as used herein refers to an immunoglobulin molecule consisting of four polypeptide chains, i.e., two heavy (H) chains and two light (L) chains which are interconnected by disulfide bonds. Each heavy chain comprises a heavy chain variable region (HCVR or VH) and a heavy chain constant region. The heavy chain constant region comprises three domains: CH1, CH2 and CH3. Each light chain comprises a light chain variable region (LCVR or VL) and a light chain constant region. The light chain constant region comprises one domain (CL1). The VH and VL regions can be further divided into hypervariable regions, termed as complementarity determining regions (CDRs), interspersed with regions that are more conserved, and termed as framework regions (FRs). Each VH and VL consists of three CDRs and four FRs, arranged in the following order from amino-terminus to carboxyl-terminus: FR1, CDR1, FR2, CDR2, FR3, CDR3, FR4. The amino acid residues of the complementarity determining region are generally, for example, residues 24-34 (LCDR1), 50-56 (LCDR2), and 89-97 (LCDR3) of the light chain variable region; and residues 31-35 (HCDR1), 50-65 (HCDR2), and 95-102 (HCDR3) of the heavy chain variable region. Kabat et al., Sequences of Proteins of Immunological Interest, 5th Ed., Public Health Service, National Institutes of Health, Bethesda, MD (1991), or for example, residues 26-32 (LCDR1), 50-52 (LCDR2), and 91-96 (LCDR3) of the light chain variable region; and residues 26-32 (HCDR1), 53-55 (HCDR2), and 96-101 (HCDR3) of the heavy chain variable region; Chothia and Lesk, J. Molecular Biology 196:901-917 (1987). The numbering convention for amino acids in this application adopts the Kabat numbering convention.

As used herein, the term "antigen-binding fragment" of an antibody, refers to one or more fragments of an antibody that retain the ability to specifically bind to an antigen (e.g., human PVRIG). It has been demonstrated that, the antigen-binding function of an antibody can be performed by certain fragments of a full-length antibody. Examples of binding fragments encompassed by the term "antigen-binding fragment" of an antibody comprise: (i) a Fab fragment, i.e., a monovalent fragment consisting of the VL, VH, CL1 and CH1 domains; (ii) a F(ab')₂ fragment, i.e., a bivalent fragment consisting of two Fab' fragments linked by disulfide bonds in the hinge region; (iii) a Fd fragment consisting of the VH and CH1 domains; (iv) a Fv fragment consisting of the single-armed VL and VH domains of an antibody; (v) a dAb fragment consisting of the VH domain (Ward et al. (1989) Nature 241:544-546); and (vi) CDRs. In addition, although the two domains of the Fv fragment, VL and VH, are encoded by different genes, they can be linked together by a synthetic linker through recombinant methods to form a single linked chain, wherein the VL and VH regions pair to form a monovalent molecule (called single-chain Fv (scFv). See, for example, Bird et al. (1988) Science 242: 423-426; and Huston et al. (1988) Proc. Natl. Acad. Sci. USA 85:5879-5883). Such single-chain antibodies are also encompassed within the term "antigen-binding fragment" of an antibody. Other forms of single chain antibodies, such as bispecific antibodies, are also included (see, e.g., Holliger et al. (1993) Proc. Natl. Acad Sci. USA 90:6444-6448).

The term "humanized antibody" comprises minimal sequence derived from non-human immunoglobulin. For the most part, humanized antibodies are human immunoglobulins (recipient antibody) in which residues from a CDR region of the recipient are replaced by residues from a CDR region (donor antibody) of a non-human species such as mouse, rat, rabbit or non-human primate having the desired specificity, affinity, and performance. In some examples, framework region (FR) residues of the human immunoglobulin are replaced by corresponding non-human residues. Furthermore, humanized antibodies may comprise residues that are not found in the recipient antibody or in the donor antibody. These modifications are intended to further refine antibody performance. In general, a humanized antibody comprises substantially all of at least one (and typically two) variable domains, wherein all or substantially all of the CDR regions correspond to those of a non-human immunoglobulin, while all or substantially all of the FR sequences are human immunoglobulin sequences. When the VH and VL sequences in a humanized antibody are all derived from non-human species, the humanized antibody is a chimeric antibody. The humanized antibody optionally also comprises at least a portion of an immunoglobulin constant region (Fc), typically that of a human immunoglobulin. For details, see Jones et al., Nature, 321: 522-525 (1986); Riechmann et al., Nature 332: 323-329 (1988); and Presta, Curr. Op.Struct. Biol. 2: 593-596 (1992).

The term "human antibody" as used herein is intended to comprise antibodies in which both the framework and CDR regions in the variable regions are derived from human germline immunoglobulin sequences. Furthermore, if the antibody comprises a constant region, then the constant region is also derived from human germline immunoglobulin sequences. The human antibodies of the application may comprise amino acid residues not encoded by human germline immunoglobulin sequences (e.g., mutations introduced by random or site-specific mutagenesis in vitro or by somatic mutation in vivo).

"Homology" is defined as the percentage of identical residues in amino acid sequence variants after aligning the sequences and introducing gaps (if necessary) to achieve the maximum percentage homology. Methods and computer programs for alignment are well known in the art.

An expression vector is a DNA molecule comprising a gene that is expressed in a host cell. Typically, gene expression is under the control of specific regulatory elements, including constitutive and inducible promoters, tissue-specific regulatory elements, and enhancers. Such a gene is called to be "operably linked to" the regulatory element.

The host cell can be any prokaryotic or eukaryotic cell that comprises an expression vector. The term also encompasses those prokaryotic or eukaryotic cells that have been genetically engineered to comprise a cloned gene in the chromosome or genome of the host cell, or in cytoplasm of the host cell, as well as transgenic animals.

An antibody-drug conjugate refers to an anti-human PVRIG antibody or an antigen-binding fragment thereof conjugated to a therapeutic agent such as cytotoxin, chemotherapeutic drug, immunosuppressant or radioactive isotope. Cytotoxin comprises any agent that damages cells. Examples of cytotoxic and chemotherapeutic agents suitable for forming immunoconjugates are well known in the art. See, e.g., WO05/103081.

A bispecific antibody is an antibody having binding specificity for at least two different epitopes. For example, a bispecific antibody can bind to two different epitopes of human PVRIG protein. Other such antibodies may combine the human PVRIG binding site with binding sites for other antigens. A bispecific antibody can be prepared as a full-length antibody or an antigen-binding fragment thereof (such as an F(ab')₂ bispecific antibody).

The phrase "therapeutically effective amount" means an amount that produces the desired effect on the subject being administered. The exact dosage will depend on the purpose of the treatment, the age and size of the subject, the route of administration, etc., and can be determined by a person skilled in the art by known techniques (e.g., see Lloyd (1999) The Art, Science and Technology of Pharmaceutical Compounding). If it is to treat cancer, the therapeutically effective amount of the drug can reduce the number of cancer cells; reduce the size of the tumor; inhibit (i.e., slow down to a certain extent, preferably stop) the infiltration of cancer cells into peripheral organs; inhibit (i.e., slow down to a certain extent, preferably stop) tumor metastasis; to a certain extent, inhibit tumor growth; and/or to a certain extent, alleviate one or more symptoms associated with cancer.

As used herein, the term "subject" comprises any human or non-human animal. The term "non-human animal" comprises all vertebrates, e.g., mammal and non-mammal, such as non-human primate, sheep, dog, cat, horse, cow, chicken, amphibian, reptile, etc.

### BRIEF DESCRIPTION OF THE DRAWINGS

Fig. 1 shows the results of the binding experiment between chimeric antibodies and soluble PVRIG.
Fig. 2 shows the results of binding experiment between humanized antibodies and soluble human PVRIG protein.
Fig. 3 shows the results of binding experiment between humanized antibodies and soluble cynomolgus monkey PVRIG.
Fig. 4 shows the results of binding experiment between a humanized antibody and PVRIG on the surface of cells overexpressing human PVRIG.
Fig. 5 shows the results of binding experiment between a humanized antibody and PVRIG on the surface of cells overexpressing cynomolgus monkey PVRIG.
Fig. 6 shows the results of ELISA assay on a humanized antibody blocking the binding of PVRIG protein to CD112 protein.
Fig. 7 shows the results of flow cytometry on a humanized antibody blocking the binding of PVRIG protein to CD112 cells.
Fig. 8 shows the results of reporter gene assay on a humanized antibody blocking the binding of PVRIG to CD112 cells.
Fig. 9A shows the results of a chimeric antibody inhibiting the volume changes of a transplanted tumor in A375 tumor-bearing mice.
Fig. 9B shows the results of the effect of a chimeric antibody on the body weight of A375 tumor-bearing mice.
Fig. 9C shows the results of a chimeric antibody inhibiting the volume changes of individual tumors transplanted in A375 tumor-bearing mice.
Fig. 10 shows the effect of an anti-PVRIG monoclonal antibody and a combination of it with an anti-PD-1 monoclonal antibody on the volume of human melanoma A375 mixed with PBMC subcutaneous transplanted tumor.
Fig. 11 Drug concentration-time curves of cynomolgus monkeys after a single intravenous injection of an anti-PVRIG monoclonal antibody.

### DETAILED DESCRIPTION

### Example 1: Acquisition of Anti-human PVRIG Monoclonal Antibodies

Healthy BALB/c mice were immunized with recombinant human PVRIG extracellular domain protein (whose amino acid sequence is shown as SEQ ID NO: 51). After the initial immunization, booster immunizations were performed every 14 days for a total of 4 times. Serum titers of the mice were detected by flow cytometry, and mice with high serum antibody titers were selected for cell fusion. PVRIG extracellular domain protein was injected by tail vein for ictus immunization 3 days before fusion. On the day of fusion, mice were euthanized, and the spleens of mice were removed under sterile conditions to prepare single-cell suspensions. SP2/0 cells and spleen cells were mixed at a ratio of 1:1 and fused by electrofusion. The fused cells were plated in a 96-well cell culture plate, with each well containing 1-3 hybridoma cells. After adding the selection medium (HAT medium), the cells were cultured in a 37°C, CO₂ incubator to screen at about 10 days depending on the cell growth conditions.

The amino acid sequence of the recombinant human PVRIG extracellular domain:

TPEVWVQVRMEATELSSFTIRCGFLGSGSISLVTVSWGGPNGAGGTTLAVLHPERGIRQWAP
ARQARWETQSSISLILEGSGASSPCANTTFCCKFASFPEGSWEACGSLPPSSDPGLSAPPTPAP
ILRAD (SEQ ID NO: 51).

High-affinity antibodies were selected to obtain the heavy and light chain variable region fragments of mouse immunoglobulins through RNA extraction, reverse transcription and PCR amplification, and then performed sequencing. The amino acid sequences of the heavy chain and light chain CDR regions and variable regions of clones 1B10E8, 21F10C10 and 43F7C9 were obtained finally, and shown as follows:
1B10E8:
   Heavy chain CDR1: SYWMH (SEQ ID NO: 1)
   Heavy chain CDR2: EINPSNGLTNYNEQFRN (SEQ ID NO: 2) Heavy chain CDR3: HHGSTYYALDS (SEQ ID NO: 3)
   Heavy chain variable region sequence: 
QVQLQQPGAELVKPGASVKLSCKASGYSFPSYWMHWVRQRPGQDLEWIGEINPSNGLTNY
NEQFRNKATLTVDKSSSTAYMQLSSLTSEDSAVYYCARHHGSTYYALDSWGQGTSITVSS
(SEQ ID NO: 7);
1B10E8:
   Light chain CDR1: KSSQSLLNSINQKNFLA (SEQ ID NO: 4)
   Light chain CDR2: FASTRES (SEQ ID NO: 5)
   Light chain CDR3: QQHYSTPPT (SEQ ID NO: 6);
   Light chain variable region sequence: 
DIVMTQSPSSLAMSVGQKVTMSCKSSQSLLNSINQKNFLAWYQQRPGQSPKLLVYFASTRE
SGVPDRFIGSGSGTDFTLTISSVQAEDLADYFCQQHYSTPPTFGGGTKLEIK (SEQ ID NO: 8);
21F10C10:
   Heavy chain CDR1: NNFIE (SEQ ID NO: 17)
   Heavy chain CDR2: VINPGSGDANYNEKFKG (SEQ ID NO: 18)
   Heavy chain CDR3: HWLAY (SEQ ID NO: 21);
   Heavy chain variable region sequence: 
QVQLQQSGAELVRPGTSVKVSCKASGYAFINNFIEWVKQRPGQGLEWIGVINPGSGDANYN
EKFKGKATLTADKSSTTAYIQLSSLTSDDSAVYFCASHWLAYWGQGTLVTVSA (SEQ ID NO:
27);
21F10C10:
   Light chain CDR1: KSSQSLLSSGNQKNYLA (SEQ ID NO: 22)
   Light chain CDR2: GASTRES (SEQ ID NO: 24)
   Light chain CDR3: QNGHFYPYT (SEQ ID NO: 26);
   Light chain variable region sequence: 
DIVMTQSPSSLSVSAGEKVTMSCKSSQSLLSSGNQKNYLAWYQQKPGQPPKLLIYGASTRE
SGVPDRFTGSGSGTDFTLTISSVQAEDLAVYYCQNGHFYPYTFGGGTKLEIK (SEQ ID NO:
28);
43H7C9:
   Heavy chain CDR1: SDYAWN (SEQ ID NO: 36)
   Heavy chain CDR2: YIRYNGNTNYNPSLKS (SEQ ID NO: 37)
   Heavy chain CDR3: IYYDYDGFVY (SEQ ID NO: 38);
   Heavy chain variable region sequence: 
DVQLQESGPGLVKPSQSLSLTCTVTGYSITSDYAWNWIRQFPGNRLEWMGYIRYNGNTNYN
PSLKSRISITRDTSKNQFFLQLNSVTTEDTATYYCAIIYYDYDGFVYWGQGTLVTVSA (SEQ
ID NO: 42);
43H7C9:
   Light chain CDR1: RASQNVYENLH (SEQ ID NO: 39)
   Light chain CDR2: FASDSIS (SEQ ID NO: 40)
   Light chain CDR3: LQLYSTPYT (SEQ ID NO: 41);
   Light chain variable region sequence:

DILLTQSPATLSVTPGETVSLSCRASQNVYENLHWYQQKSHRSPRLLIKFASDSISGIPSRFTG
SGSGTDYTLSINSVKPEDEGIYYCLQLYSTPYTFGGGTELEIK (SEQ ID NO: 43).

Note: In the above antibody sequences, the underlined sequences are CDR sequences (determined and annotated according to the Kabat numbering system).

### Example 2: Detecting the Affinity of Chimeric Antibodies by ELISA Assay

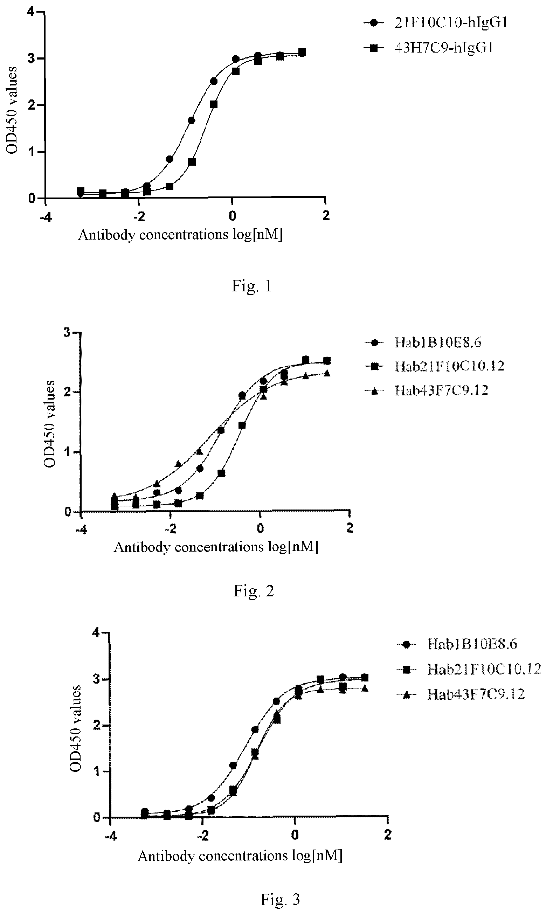

According to the variable region sequence information of the encoding gene of the above monoclonal antibody, an expression vector of human-mouse chimeric antibody was constructed. VH and VL were respectively constructed into a eukaryotic expression vector comprising hIgG1-kappa constant region. The obtained eukaryotic expression vector was transiently transfected into HEK-293 cells and cultured for 6 days. The culture supernatant was harvested and purified by Protein A to obtain target antibodies, which were named 21F10C10-hIgG1 and 43F7C9-hIgG1, respectively. The affinity of the antibodies to the recombinant PVRIG protein was determined by ELISA. The PVRIG extracellular full-length protein diluted to a concentration of 500 ng/mlwas coated on the ELISA plate. The plate was washed 3 times with PBST buffer, and then 200 µl of PBS buffer containing 2% BSA was added, and the plate was blocked at 37°C for 2h. After the blocking was completed, the plate was washed 3 times with PBST buffer; 3-fold gradient dilutions of the test antibody (0-33.3 nM) were added, incubated at 37°C for 2 h; then the plate was washed 3 times with PBST buffer. Horseradish peroxidase-labeled goat anti-human antibody was added, incubated at 37°C for 1h; then the plate was washed three times with PBST buffer. TMB single-component color developing solution was added for color development, reading the absorbance value after adding the stop solution. The obtained results of affinity are shown in Fig. 1 and Table 1.

**Table 1: EC₅₀ values of chimeric antibody affinity**

| Antibody names | EC₅₀ values (nM) |
|---|---|
| 21F10C10- hIgG1 | 0.1222 |
| 43F7C9- hIgG1 | 0.2901 |

The EC₅₀ values of 21F10C10-hIgG1 and 43F7C9-hIgG1 binding to PVRIG protein were 0.1222nM and 0.2901nM, respectively.

### Example 3: Antibody Humanization

Homology modeling was performed by Discovery Studio and Schrödinger Antibody Modeling. Through structural simulation and rational design, a human framework region closest to the mouse antibody framework region was obtained.

The CDRs of the light chain and heavy chain were transplanted onto the framework sequences of the corresponding light chain and heavy chain genes, respectively, so as to obtain humanized antibodies Hab1B10E8, Hab21F10C10 and Hab43F7C9. The 3D model was then constructed by Discovery Studio and Schrödinger Antibody Modeling to analyze whether there were any framework positions where substitution of mouse amino acids for human amino acids affected binding and/or CDR conformation, and reverse mutations were performed. The sequences are shown in Tables 2-3 (in Tables 2-3, the CDR region numbering rules all adopt the Kabat numbering rules). The obtained reverse mutations were expressed in combination, and the expressed antibody combinations are shown in Tables 4-6.

**Table 2: Sequences with VH reverse mutations (CDR numbering is kabat)**

| Constructs | Mutants |
|---|---|
| HabIB10E8-VH.1a | Heavy chain CDR1: SYWMH (SEQ ID NO: 1) |
| | Heavy chain CDR2: EINPSNGLTNYNEQFRN (SEQ ID NO: 2) |
| | Heavy chain CDR3: HHGSTYYALDS (SEQ ID NO: 3) |
| | QVQLVQSGAEVKKPGASVKVSCKASGYSFPSYWMHWVRQAPGQG LEWMGEINPSNGLTNYNEQFRNRVTMTRDTSTSTVYMELSSLRSED TAVYYCARHHGSTYYALDSWGQGTLVTVSS (SEQ ID NO: 9) |
| Hab1B10E8-VH.1b | Heavy chain CDR1: SYWMH (SEQ ID NO: 1) |
| | Heavy chain CDR2: EINPSNGLTNYNEQFRN (SEQ ID NO: 2) |
| | Heavy chain CDR3: HHGSTYYALDS (SEQ ID NO: 3) |
| | QVQLVQSGAEVKKPGASVKVSCKASGYSFPSYWMHWVRQAPGQG LEWIGEINPSNGLTNYNEQFRNRVTMTVDTSTSTVYMELSSLRSEDT AVYYCARHHGSTYYALDSWGQGTLVTVSS (SEQ ID NO: 10) |
| Hab1B10E8-VH.1c | Heavy chain CDR1: SYWMH (SEQ ID NO: 1) |
| | Heavy chain CDR2: EINPSNGLTNYNEQFRN (SEQ ID NO: 2) |
| | Heavy chain CDR3: HHGSTYYALDS (SEQ ID NO: 3) |
| | QVQLVQSGAEVKKPGASVKVSCKASGYSFPSYWMHWVRQAPGQG LEWIGEINPSNGLTNYNEQFRNRATMTVDTSTSTVYMELSSLRSEDT AVYYCARHHGSTYYALDSWGQGTLVTVSS (SEQ ID NO: 11) |
| Hab1B10E8-VH.1d | Heavy chain CDR1: SYWMH (SEQ ID NO: 1) |
| | Heavy chain CDR2: EINPSNGLTNYNEQFRN (SEQ ID NO: 2) |
| | Heavy chain CDR3: HHGSTYYALDS (SEQ ID NO: 3) |
| | QVQLVQSGAEVKKPGASVKVSCKASGYSFPSYWMHWVRQAPGQG LEWIGEINPSNGLTNYNEQFRNRATMTVDTSSSTVYMELSSLRSEDT AVYYCARHHGSTYYALDSWGQGTLVTVSS (SEQ ID NO: 12) |
| Hab21F10C10-VH. | Heavy chain CDR1: NNFIE (SEQ ID NO: 17) |
| 1a | Heavy chain CDR2: VINPGSGDANYNEKFKG (SEQ ID NO: 18) |
| | Heavy chain CDR3: HWLAY (SEQ ID NO: 21) |
| | QVQLVQSGAEVKKPGASVKVSCKASGYAFINNFIEWVRQAPGQGLE WIGVINPGSGDANYNEKFKGRATLTADKSITTAYIELSRLRSDDTAVY YCASHWLAYWGQGTTVTVSS (SEQ ID NO: 29) |
| Hab21F10C10-VH. | Heavy chain CDR1: NNFIE (SEQ ID NO: 17) |
| 1b | Heavy chain CDR2: VINPGSGDANYNEKFQG (SEQ ID NO: 19) |
| | Heavy chain CDR3: HWLAY (SEQ ID NO: 21) |
| | QVQLVQSGAEVKKPGASVKVSCKASGYAFINNFIEWVRQAPGQGLE WIGVINPGSGDANYNEKFQGRVTLTADKSISTAYIELSRLRSDDTAVY YCASHWLAYWGQGTTVTVSS (SEQ ID NO: 30) |
| Hab21F10C10-VH. | Heavy chain CDR1: NNFIE (SEQ ID NO: 17) |
| 1c | Heavy chain CDR2: VINPGSGDANYNEKFQG (SEQ ID NO: 19) |
| | Heavy chain CDR3: HWLAY (SEQ ID NO: 21) |
| | QVQLVQSGAEVKKPGASVKVSCKASGYAFINNFIEWVRQAPGQGLE WMGVINPGSGDANYNEKFQGRVTMTADTSISTAYMELSRLRSDDTA VYYCASHWLAYWGQGTTVTVSS (SEQ ID NO: 31) |
| Hab21F10C10-VH. | Heavy chain CDR1: NNFIE (SEQ ID NO: 17) |
| 1d | Heavy chain CDR2: VINPGSGDANYAQKFQG (SEQ ID NO: 20) |
| | Heavy chain CDR3: HWLAY (SEQ ID NO: 21) |
| | QVQLVQSGAEVKKPGASVKVSCKASGYTFINNFIEWVRQAPGQGLE WMGVINPGSGDANYAQKFQGRVTMTRDTSISTAYMELSRLRSDDTA VYYCASHWLAYWGQGTTVTVSS (SEQ ID NO: 32) |
| Hab43F7C9-VH.1a | Heavy chain CDR1: SDYAWN (SEQ ID NO: 36) |
| | Heavy chain CDR2: YIRYNGNTNYNPSLKS (SEQ ID NO: 37) |
| | Heavy chain CDR3: IYYDYDGFVY (SEQ ID NO: 38) |
| | DVQLQESGPLVKPSETLSLTCTVTGYSITSDYAWNWIRQFPGKGLEW MGYIRYNGNTNYNPSLKSRITISRDTSKNQFSLKLSSVTAADTAVYY CAIIYYDYDGFVYWGQGTTVTVSS (SEQ ID NO: 44) |
| Hab43F7C9-VH.1b | Heavy chain CDR1: SDYAWN (SEQ ID NO: 36) |
| | Heavy chain CDR2: YIRYNGNTNYNPSLKS (SEQ ID NO: 37) |
| | Heavy chain CDR3: IYYDYDGFVY (SEQ ID NO: 38) |
| | QVQLQESGPGLVKPSETLSLTCTVTGYSITSDYAWNWIRQPPGKGLE WMGYIRYNGNTNYNPSLKSRVTISVDTSKNQFSLKLSSVTAADTAV YYCAIIYYDYDGFVYWGQGTTVTVSS (SEQ ID NO: 45) |
| Hab43F7C9-VH.1c | Heavy chain CDR1: SDYAWN (SEQ ID NO: 36) |
| | Heavy chain CDR2: YIRYNGNTNYNPSLKS (SEQ ID NO: 37) |
| | Heavy chain CDR3: IYYDYDGFVY (SEQ ID NO: 38) |
| | QVQLQESGPGLVKPSETLSLTCTVSGYSITSDYAWNWIRQPPGKGLE WIGYIRYNGNTNYNPSLKSRVTISVDTSKNQFSLKLSSVTAADTAVY YCAIIYYDYDGFVYWGQGTTVTVSS (SEQ ID NO: 46) |
| Hab43F7C9-VH.1d | Heavy chain CDR1: SDYAWN (SEQ ID NO: 36) |
| | Heavy chain CDR2: YIRYNGNTNYNPSLKS (SEQ ID NO: 37) |
| | Heavy chain CDR3: IYYDYDGFVY (SEQ ID NO: 38) |
| | QVQLQESGPGLVKPSETLSLTCTVSGYSISSDYAWNWIRQPPGKGLE WIGYIRYNGNTNYNPSLKSRVTISVDTSKNQFSLKLSSVTAADTAVY YCAIIYYDYDGFVYWGQGTTVTVSS (SEQ ID NO: 47) |

**Table 3: Sequences with VL reverse mutations**

| Constructs | Mutants |
|---|---|
| Hab1B10E8-VL.1a | Light chain CDR1: KSSQSLLNSINQKNFLA (SEQ ID NO: 4) |
| | Light chain CDR2: FASTRES (SEQ ID NO: 5) |
| | Light chain CDR3: QQHYSTPPT (SEQ ID NO: 6) |
| | DIVMTQSPDSLAVSLGERATINCKSSQSLLNSINQKNFLAWYQQKP GQPPKLLIYFASTRESGVPDRFSGSGSGTDFTLTISSLQAEDVAVYY CQQHYSTPPTFGGGTKLEIK (SEQ ID NO: 13) |
| Hab1B10E8-VL.1b | Light chain CDR1: KSSQSLLNSINQKNFLA (SEQ ID NO: 4) |
| | Light chain CDR2: FASTRES (SEQ ID NO: 5) |
| | Light chain CDR3: QQHYSTPPT (SEQ ID NO: 6) |
| | DIVMTQSPDSLAVSLGERATINCKSSQSLLNSINQKNFLAWYQQKP GQPPKLLVYFASTRESGVPDRFSGSGSGTDFTLTISSLQAEDVAVYF CQQHYSTPPTFGGGTKLEIK (SEQ ID NO: 14) |
| Hab1B10E8-VL.1c | Light chain CDR1: KSSQSLLNSINQKNFLA (SEQ ID NO: 4) |
| | Light chain CDR2: FASTRES (SEQ ID NO: 5) |
| | Light chain CDR3: QQHYSTPPT (SEQ ID NO: 6) |
| | DIVMTQSPDSLAVSLGERATINCKSSQSLLNSINQKNFLAWYQQRP GQPPKLLVYFASTRESGVPDRFSGSGSGTDFTLTISSLQAEDVAVYF CQQHYSTPPTFGGGTKLEIK (SEQ ID NO: 15) |
| Hab1B10E8-VL.1d | Light chain CDR1: KSSQSLLNSINQKNFLA (SEQ ID NO: 4) |
| | Light chain CDR2: FASTRES (SEQ ID NO: 5) |
| | Light chain CDR3: QQHYSTPPT (SEQ ID NO: 6) |
| | DIVMTQSPDSLAVSLGERATMSCKSSQSLLNSINQKNFLAWYQQRP GQPPKLLVYFASTRESGVPDRFSGSGSGTDFTLTISSLQAEDVAVYF CQQHYSTPPTFGGGTKLEIK (SEQ ID NO: 16) |
| Hab21F10C10-VL.1 a | Light chain CDR1: KSSQSLLSSGNQKNYLA (SEQ ID NO: 22) |
| | Light chain CDR2: GASTRES (SEQ ID NO: 24) |
| | Light chain CDR3: QNGHFYPYT (SEQ ID NO: 26) |
| | EIVMTQSPPTLSVSPGERVTMSCKSSQSLLSGNQKNYLAWYQQK PGQAPRLLIYGASTRESGVPDRFSGSGSGTDFTLTISSVQPEDFAVY YCQNGHFYPYTFGGGTKLEIK (SEQ ID NO: 33) |
| Hab21F10C10-VL.1 | Light chain CDR1: KSSQSLLSSGNQKNYLA (SEQ ID NO: 22) |
| b | Light chain CDR2: GASTRES (SEQ ID NO: 24) |
| | Light chain CDR3: QNGHFYPYT (SEQ ID NO: 26) |
| | EIVMTQSPPTLSLSPGERVTLSCKSSQSLLSSGNQKNYLAWYQQKP GQAPRLLIYGASTRESGIPARFSGSGSGTDFTLTISSLQPEDFAVYYC QNGHFYPYTFGGGTKLEIK (SEQ ID NO: 34) |
| Hab21F10C10-VL.1 | Light chain CDR1: RSSQSLLSSGNQKNYLS (SEQ ID NO: 23) |
| c | Light chain CDR2: GASTRET (SEQ ID NO: 25) |
| | Light chain CDR3: QNGHFYPYT (SEQ ID NO: 26) |
| | EIVMTQSPPTLSLSPGERVTLSCRSSQSLLSSGNQKNYLSWYQQKP GQAPRLLIYGASTRETGIPARFSGSGSGTDFTLTISSLQPEDFAVYYC QNGHFYPYTFGGGTKLEIK (SEQ ID NO: 35) |
| Hab43F7C9-VL.1a | Light chain CDR1: RASQNVYENLH (SEQ ID NO: 39) |
| | Light chain CDR2: FASDSIS (SEQ ID NO: 40) |
| | Light chain CDR3: LQLYSTPYT (SEQ ID NO: 41) |
| | DIQLTQSPSSLSVSVGDRVTLTCRASQNVYENLHWYQQKPGKAPR LLIKFASDSISGIPSRFSGSGSGTDYTLTI SSVQPEDFATYYCLQLYSTPYTFGGGTKLEIK (SEQ ID NO: 48) |
| Hab43F7C9-VL.1b | Light chain CDR1: RASQNVYENLH (SEQ ID NO: 39) |
| | Light chain CDR2: FASDSIS (SEQ ID NO: 40) |
| | Light chain CDR3: LQLYSTPYT (SEQ ID NO: 41) |
| | DIQLTQSPSSLSASVGDRVTITCRASQNVYENLHWYQQKPGKAPK LLIKFASDSISGIPSRFSGSGSGTDYTLTISSLQPEDFATYYCLQLYST PYTFGGGTKLEIK (SEQ ID NO: 49) |
| Hab43F7C9-VL.1c | Light chain CDR1: RASQNVYENLH (SEQ ID NO: 39) |
| | Light chain CDR2: FASDSIS (SEQ ID NO: 40) |
| | Light chain CDR3: LQLYSTPYT (SEQ ID NO: 41) |
| | DIQMTQSPSSLSASVGDRVTITCRASQNVYENLHWYQQKPGKAPK LLIKFASDSISGVPSRFSGSGSGTDFTLTISSLQPEDFATYYCLQLYST PYTFGGGTKLEIK (SEQ ID NO: 50) |

**Table 4: Schematic structures of 1B10E8 humanized antibody combinations**

| | Hab1B10E8-VL .1a | Hab1B10E8-VL .1b | Hab1B10E8-VL .1c | Hab1B10E8-VL .1d |
|---|---|---|---|---|
| Hab1B10E8-VH.1a | Hab1B10ES8.1 | Hab1B10E8.2 | Hab1B10E8.3 | Hab1B10E8.4 |
| Hab1B10E8-VH.1b | Hab1B10E8.5 | Hab1B10E8.6 | Hab1B10E8.7 | Hab1B10E8.8 |
| Hab1B10E8-VH.1c | Hab1B10E8.9 | Hab1B10E8.10 | Hab1B10E8.11 | Hab1B10E8.12 |
| Hab1B10E8-VH.1d | Hab1B10E8.13 | Hab1B10E8.14 | Hab1B10E8.15 | Hab1B10E8.16 |

**Table 5: Schematic structures of 21F10C10 humanized antibody combinations**

| | Hab21F10C10-VL.1a | Hab21F10C10-VL.1b | Hab21F10C10-VL.1c |
|---|---|---|---|
| Hab21F10C10-VH.1a | Hab21F10C10.1 | Hab21F10C10.2 | Hab21F10C10.3 |
| Hab21F10C10-VH.1b | Hab21F10C10.4 | Hab21F10C10.5 | Hab21F10C10.6 |
| Hab21F10C10-VH.1c | Hab21F10C10.7 | Hab21F10C10.8 | Hab21F10C10.9 |
| Hab21F10C10-VH.1d | Hab21F10C10.10 | Hab21F10C10.11 | Hab21F10C10.12 |

**Table 6: Schematic structures of 43F7C9 humanized antibody combinations**

| | Hab43F7C9-VL.1a | Hab43F7C9-VL.1b | Hab43F7C9-VL.1c |
|---|---|---|---|
| Hab43F7C9-VH.1a | Hab43F7C9.1 | Hab43F7C9.2 | Hab43F7C9.3 |
| Hab43F7C9-VH.1b | Hab43F7C9.4 | Hab43F7C9.5 | Hab43F7C9.6 |
| Hab43F7C9-VH.1c | Hab43F7C9.7 | Hab43F7C9.8 | Hab43F7C9.9 |
| Hab43F7C9-VH.1d | Hab43F7C9.10 | Hab43F7C9.11 | Hab43F7C9.12 |

The constant region sequences of the antibodies used in the Examples are as follows:
IgG1 constant region 
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK (SEQ ID NO: 51);
IgG4 constant region 
ASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRVESKYGPPCPSCPAPEFLGGPSVFLFPPK
PKDTLMISRTPEVTCVVVDVSQEDPEVQFNWYVDGVEVHNAKTKPREEQFNSTYRVVSVL
TVLHQDWLNGKEYKCKVSNKGLPSSIEKTISKAKGQPREPQVYTLPPSQEEMTKNQVSLTC
LVKGFYPSDIAVEWESNGQPENNYKTTPPVLDSDGSFFLYSRLTVDKSRWQEGNVFSCSVM
HEALHNHYTQKSLSLSLGK (SEQ ID NO: 52);
IgG1 constant region, where Fc comprises S239D-A330L-I332E mutations, referred to as DLE mutation

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPDVF
LFPPKPKDTLMISRTPEVTCVVVDVSHEDPEVKFNWYVDGVEVHNAKTKPREEQYNSTYR
VVSVLTVLHQDWLNGKEYKCKVSNKALPLPEEKTISKAKGQPREPQVYTLPPSRDELTKNQ
VSLTCLVKGFYPSDIAVEWESNGQPENNYKTTPPVLDSDGSFFLYSKLTVDKSRWQQGNVF
SCSVMHEALHNHYTQKSLSLSPGK (SEQ ID NO: 53).

### Example 4: Detecting Affinity of Humanized Anti-PVRIG Antibodies

### 4.1 The affinity of humanized antibody to human PVRIG protein was detected by Fortebio

The HIS1K biosensor was used, and human PVRIG-His protein was immobilized on the biosensor to a binding response threshold of 0.3 nm. After a 120 s baseline step, the sensor was immersed in an anti-PVRIG antibody diluted with 0.02% PBST buffer, with a starting antibody concentration of 25 nM, performing gradient dilution of 2-fold concentration with 7 gradients set. The binding and dissociation time of the immobilized substance and the analyte were 120 s and 300 s, respectively. The affinity data of antigen and antibody was calculated by regression analysis software based on the binding curve. Among all the tested antibodies, Hab1B10E8.6, Hab21F10C10.12 and Hab43F7C9.12 showed the highest affinity and fewer reverse mutations. The results are shown in Table 7.

**Table 7: Affinity of humanized antibody to human PVRIG protein was detected by Fortebio**

| Samples | Ka(1/Ms) | Kd(1/s) | KD(M) |
|---|---|---|---|
| Hab1B10E8.6 | 7.82E+05 | <1.0E-07 | <1.0E-12 |
| Hab21F10C10.12 | 8.37E+05 | <1.0E-07 | <1.0E-12 |
| Hab43F7C9.12 | 9.63E+05 | <1.0E-07 | <1.0E-12 |

### 4.2 The affinity of humanized antibody to human PVRIG protein was detected by ELISA

The affinity of humanized antibody to recombinant human PVRIG protein was determined by ELISA. The PVRIG protein was diluted to 500 ng/ml, and coated on the ELISA plate. After blocking, 3-fold gradient dilution of antibody (0-66.7 nM) was added, then performed color development and reading after incubation with goat anti-human secondary antibody. The affinity results obtained after GraphPad fitting are shown in Fig. 2 and Table 8.

**Table 8: EC₅₀ values of humanized antibody affinity detected by ELISA**

| Antibody names | EC₅₀ values (nM) |
|---|---|
| Hab1B10E8.6 | 0.0869 |
| Hab21F10C10.12 | 0.1646 |
| Hab43F7C9.12 | 0.1447 |

The EC₅₀ values of Hab1B10E8.6, Hab21F10C10.12 and Hab43F7C9.12 binding to human PVRIG protein were 0.0869nM, 0.1646nM and 0.1447nM, respectively.

### 4.3 The affinity of humanized antibody to cynomolgus monkey PVRIG protein was detected by ELISA

The affinity of humanized antibody to recombinant cynomolgus monkey (Cyno) PVRIG protein was determined by ELISA. The Cyno-PVRIG protein was diluted to 500 ng/ml, and coated on the ELISA plate. After blocking, a 3-fold gradient dilution of the antibody (0-33.3 nM) was added. After incubation with goat anti-human secondary antibody for 1 hour, the plate was washed and the color was developed to read. The affinity results obtained after GraphPad fitting are shown in Fig. 3 and Table 9.

**Table 9: EC₅₀ values of the affinity of the humanized antibodies to Cyno-PVRIG protein detected by ELISA**

| Antibody names | EC₅₀ values (nM) |
|---|---|
| Hab1B10E8.6 | 0.1410 |
| Hab21F10C10.12 | 0.3638 |
| Hab43F7C9.12 | 0.07967 |

The EC₅₀ values of antibodies Hab1B10E8.6, Hab21F10C10.12 and Hab43F7C9.12 binding to Cyno-PVRIG protein were not much different from those of the antibodies to human PVRIG protein.

### Example 5: Binding of Humanized Antibodies to Cells Overexpressing Human PVRIG

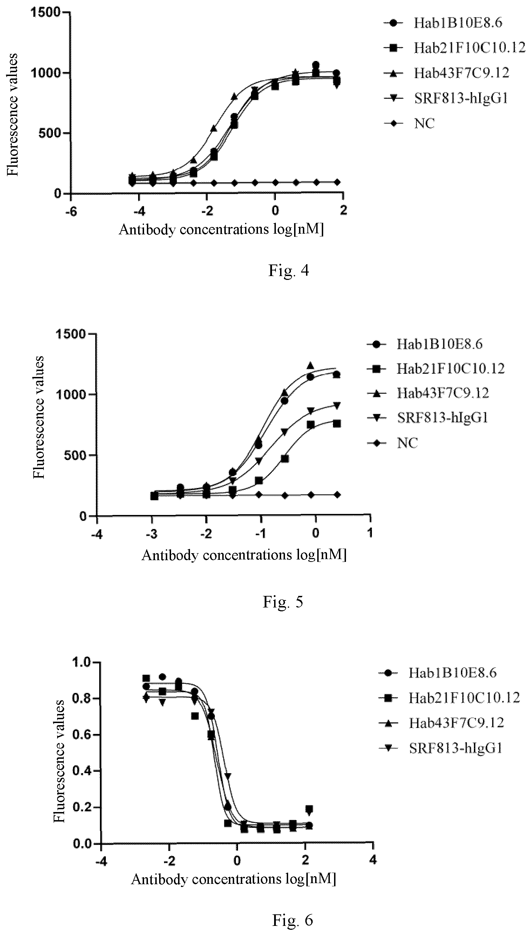

After expressing antibodies Hab1B10E8.6, Hab21F10C10.12, Hab43F7C9.12 and SRF813 (the CDR region sequence is from clone 46 of US20210253699A1, the amino acid sequence of HV is shown as SEQ ID NO: 912 in the patent application, and the amino acid sequence of LV is shown as SEQ ID NO: 918 in the patent application), the binding ability of the humanized antibodies to PVRIG on cell surface was evaluated by using the Jurkat-hPVRIG cell line overexpressing human PVRIG. The cells were inoculated at a density of 2×10⁶/ml in 96-well plate, 100 µl/well. 4-fold gradient dilutions of antibodies (0-66.7 nM) were added to incubate at 4°C for 1 h. Goat anti-human secondary antibody was added, and the cells were labeled in the dark at 4°C for 45 min. After the cells were collected and washed, the fluorescence intensity of the cells was read by flow cytometry. The experimental results are shown in Fig. 4 and Table 10, and the results show that the humanized antibody can bind to the PVRIG protein expressed on the cell line in a dose-dependent manner. At the same time, the affinity of Hab43F7C9.12 in recognizing cell surface PVRIG is better than that of the control molecule SRF813.

**Table 10: Experiment on the binding of humanized antibody to PVRIG on Jurkat cell surface**

| Tested samples | EC₅₀ (nM) of Jurkat cell level affinity |
|---|---|
| Hab1B10E8.6 | 0.05006 |
| Hab21F10C10.12 | 0.05530 |
| Hab43F7C9.12 | 0.01784 |
| SRF813-IgG1 | 0.04973 |

### Example 6: Binding of Humanized Anti-PVRIG Antibodies to Cells Overexpressing Cynomolgus Monkey PVRIG

In order to characterize the ability of the screened antibodies to recognize the cynomolgus monkey PVRIG molecule on the cell membrane, we constructed 293T cells that can stably express the full-length monkey PVRIG. The cells were seeded in a 96-well plate at a density of 2×10⁶, 100 µl/well, then washed three times with FACS buffer and the supernatant was discarded. Antibodies diluted with PBS buffer (0-2.5 nM) in a gradient were added to incubate at 4°C for 1 h. The cells were washed three times with FACS buffer, and then labeled with Alexa Fluor 488-labeled goat anti-human IgG antibody at 4°C in the dark for 45 min. The cells were washed three times with FACS buffer, and the fluorescence intensity of the cells was read by flow cytometry. The experimental results are shown in Fig. 5 and Table 11. The experimental results show that the humanized antibody can bind to the monkey PVRIG protein expressed on the cell line in a dose-dependent manner.

**Table 11: Experiment on the binding of humanized antibody to PVRIG on the surface of 293T-cynoPVRIG cells**

| Tested samples | EC₅₀ (nM) of Jurkat cell level affinity |
|---|---|
| Hab1B10E8.6 | 0.1241 |
| Hab21F10C10.12 | 0.2668 |
| Hab43F7C9.12 | 0.1079 |
| SRF813-IgG1 | 0.1498 |

### Example 7: Functional Verification of Anti-PVRIG Antibodies

The biological function of PVRIG antibody was verified by ELISA blocking experiment, FACS blocking experiment, and reporter gene experiment.

### 7.1 The antibodies block the binding of human PVRIG protein to human CD112 protein

The ELISA method was used to determine the ability of humanized PVRIG antibody to block the binding of human CD112 protein to human PVRIG protein. The CD112-His protein was diluted to a concentration of 1 µg/ml, and coated on the ELISA plate. A 3-fold gradient dilution of the antibody (0-133 nM) was mixed with the PVRIG-hFc protein and added to the coated ELISA plate to incubate at 37°C for 1 hour. After washing the plate, the HRP-labeled goat anti-human Fc antibody was added to incubate at 37°C for 1 hour. After washing the plate, the color developing solution was added, then adding the stop solution after 5 minutes and reading the results. The blocking results of the antibodies obtained are shown in Fig. 6 and Table 12 after GraphPad fitting. The results show that humanized antibodies can effectively block the binding of CD112 to PVRIG protein. At the same time, Hab1B10E8.6, Hab21F10C10.12 and Hab43F7C9.12 have better ELISA blocking ability than the control molecule SRF813.

**Table 12: Experiment on blocking the binding of PVRIG to CD112 protein by the humanized antibody**

| Tested samples | EC₅₀ (nM) of Jurkat cell level affinity |
|---|---|
| Hab1B10E8.6 | 0.2850 |
| Hab21F10C10.12 | 0.2262 |
| Hab43F7C9.12 | 0.2583 |
| SRF813-IgG1 | 0.4314 |

### 7.2 The antibodies block the binding of PVRIG to 293T-PVRL2 cells

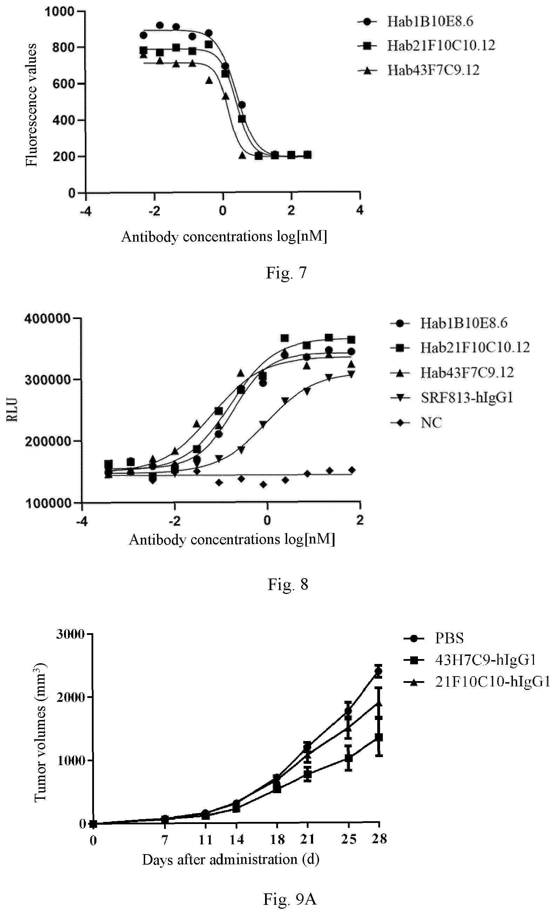

Flow cytometry was used to detect the ability of the humanized antibodies to block the binding of PVRIG protein to 293T cells overexpressing CD112. The 293T-CD112 cells at the logarithmic phase were digested and diluted to 1E6/ml, 50 µl of the cells were added to each well of the flow plate, then 25 µl of 3-fold serially diluted antibody samples (0-300 nM) and 25 µl of 1 ug/ml PVRIG-hFc protein at the same time were added to incubate at 4°C for 1 h. The cells were washed three times with FACS buffer, and then labeled with Alexa Fluor 488-labeled goat anti-human IgG antibody at 4°C in the dark for 50 min. Then the cells were washed three times with FACS buffer, and the fluorescence intensity of the cells was read by flow cytometry. The blocking results are shown in Fig. 7 and Table 13. The results show that the humanized antibodies block the binding of PVRIG to 293T-CD112 cells in a dose-dependent manner.

**Table 13: Experiment on blocking the binding of PVRIG to CD112 cells by the humanized antibodies**

| Tested samples | EC₅₀ (nM) of Jurkat cell level affinity |
|---|---|
| Hab1B10E8.6 | 2.558 |
| Hab21F10C10.12 | 2.525 |
| Hab43F7C9.12 | 1.457 |

### 7.3 Reporter gene method was used to detect blocking the binding of PVRIG to CD112 by the humanized antibodies

CD112/TCR Activator/CHO cells at the logarithmic growth phase were digested and centrifuged, and the cell density was adjusted to 4E5/ml, then inoculated into a white-walled, transparent-bottomed 96-well plate at 100 µl per well to culture at 37°C overnight. The next day, the culture supernatant was removed, and 50 µl (0-66.7 nM) of antibody samples diluted with RPMI1640 medium in a 3-fold serial dilution was added, and 50 µl of effector cells stably expressing PVRIG at the logarithmic growth phase were added at a cell density of 4E5/ml. The cells were cultured in a 37°C incubator for 6 hours, and detected by the Bright-Glo^{™} Luciferase Detection System. The reporter gene results are shown in Fig. 8 and Table 14; the results show that the humanized antibody can effectively block the binding of CD112 cells to PVRIG cells. At the same time, the ELISA blocking ability of Hab1B10E8.6, Hab21F10C10.12 and Hab43F7C9.12 is significantly better than that of the control molecule SRF813-IgG1.

**Table 14: Experiment on blocking the binding of PVRIG to PVRL2 cells by the humanized antibodies**

| Tested samples | EC₅₀ (nM) of reporter genes |
|---|---|
| Hab1B10E8.6 | 0.1841 |
| Hab21F10C10.12 | 0.1642 |
| Hab43F7C9.12 | 0.0651 |
| SRF813-IgG1 | 0.9627 |

### Example 8: Biological Activity of Anti-PVRIG Chimeric Antibodies in Animals

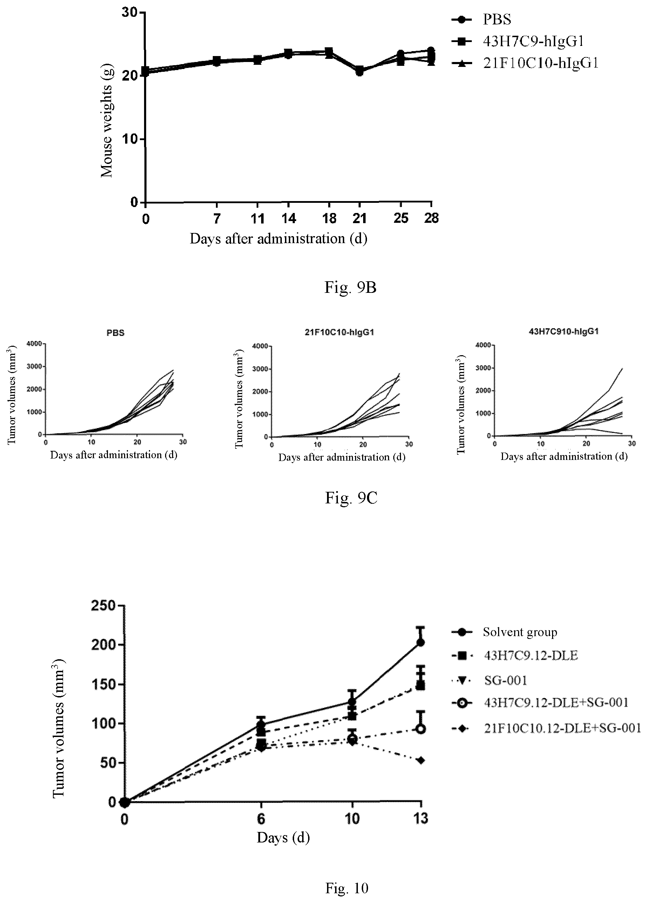

The monotherapy efficacy of 21F10C10-hIgG1, or 43H7C9-hIgG1 chimeric antibody was evaluated in a tumor xenograft model. 4×10⁶ A375 melanoma cells and 5×10⁵ human PBMCs were mixed and inoculated with Matrigel at a 1:1 ratio into NCG immunodeficient mice. On the day of transplantation, 21F10C10, 43H7C9 chimeric antibodies and PBS control were respectively injected intraperitoneally at 20 mg/kg, twice a week. In the control group, the average tumor volume was 2387.34±95.79 mm³ (mean±SE) on day 28 of treatment. Compared with the control group, the 43H7C9 group had reduced tumor growth, resulting in 43.93% tumor inhibition and a tumor volume of 1338.48±296.09 mm³; and the 21F10C10 group had reduced tumor growth, resulting in 20.83% tumor inhibition and a tumor volume of 1890.09±568.21 mm³ (Fig. 9A). The changes in body weight of each experimental group were not significant (Fig. 9B), indicating that the treatment was well tolerated. The results of individual tumor volumes are shown in Fig. 9C (multiple lines in Fig. 9C represent multiple mice).

### Example 9: Biological Activity of Anti-PVRIG Humanized Antibodies in Animals

The efficacy of humanized antibodies Hab21F10C10.12-DLE (wherein Fc comprises DLE mutations, and the constant region sequence is shown as SEQ ID NO: 53) and 43H7C9.12-DLE (wherein Fc comprises DLE mutations, and the constant region sequence is shown as SEQ ID NO: 53) was evaluated in a tumor xenograft model. After NCG mice arrived at the facility, they were adaptively fed for at least 2 days. The frozen human PBMCs were revived and counted in vitro, and then co-cultured with A375 melanoma cells treated with Mitomycin C. After co-culture, PBMCs were collected to mix with fresh A375 melanoma cells at the exponential growth phase, then resuspended in PBS to an appropriate concentration, and inoculated subcutaneously in NCG mice. On the day of transplantation, mice were randomly divided into groups according to their body weight, and the anti-PVRIG monoclonal antibody described above was intraperitoneally administered at 20 mg/kg, once every other day; and the tumor volume was measured. In the control group, the average tumor volume was 201.9±53.6 (mean±SE) on day 13 of treatment. Compared with the control group, the 43H7C9.12-DLE group had reduced tumor growth, resulting in 27.3% tumor inhibition and a tumor volume of 146.8±45.0. The 43H7C9.12-DLE+SG-001 (a PD-1 antibody, the sequence is shown below) group resulted in 61.5% tumor inhibition and a tumor volume of 77.8±52.6. The 21F10C10.12-DLE+SG-001 group resulted in 74.2% tumor inhibition and a tumor volume of 52.2±10.1 (Fig. 10).
SG-001 heavy chain: 
QVQLVESGGGVVQPGRSLRLTCKASGLTFSSSGMHWVRQAPGKGLEWVAVIWYDGSKRYY
ADSVKGRFTISRDNSKNTLFLQMNSLRAEDTAVYYCATNNDYWGQGTLVTVSSASTKGPS
VFPLAPCSRSTSESTAALGCLVKDYFPEPVTVSWNSGALTSGVHTFPAVLQSSGLYSLSSVVT
VPSSSLGTKTYTCNVDHKPSNTKVDKRVESKYGPPCPPCPAPEFLGGPSVFLFPPKPKDTLM
ISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSVMHEALHN
HYTQKSLSLSLGK (SEQ ID NO: 54);
SG-001 light chain:

EIVLTQSPATLSLSPGERATLSCRASQSVSSYLAWYQQKPGQAPRLLIYTASNRATGIPARFSG
SGSGTDFTLTISSLEPEDFAVYYCQQYSNWPRTFGQGTKVEIKRTVAAPSVFIFPPSDEQLKSG

TASVVCLLNNFYPREAKVQWKVDNALQSGNSQESVTEQDSKDSTYSLSSTLTLSKADYEK
HKVYACEVTHQGLSSPVTKSFNRGEC (SEQ ID NO: 55).

### Example 10: Pharmacokinetic Characteristics of Anti-PVRIG Humanized Antibodies

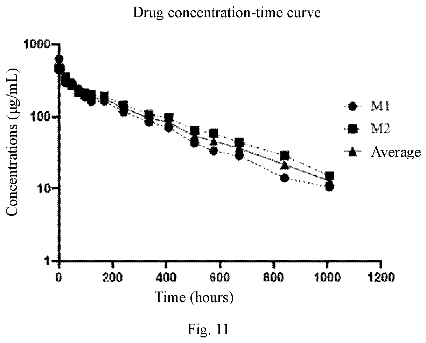

The pharmacokinetic characteristics of anti-PVRIG monoclonal antibody in cynomolgus monkeys were investigated by detecting the drug concentration in serum after a single intravenous infusion of 43H7C9.12-DLE humanized antibody (20 mg/kg) in cynomolgus monkeys. Two male cynomolgus monkeys were intravenously infused with 20 mg/kg of anti-PVRIG monoclonal antibody, and whole blood samples were collected before and after the drug administration, and the serum was separated. The ELISA method was used to detect the concentration of anti-PVRIG monoclonal antibody in serum samples and obtain the PK parameters. At this dose, the half-life was approximately 227 hours. The results are shown in Fig. 11 and Table 15, wherein M1 and M2 represent two cynomolgus monkeys.

**Table 15: PK parameters after single intravenous infusion of anti-PVRIG monoclonal antibody in cynomolgus monkeys**

| Animals | t_{1/2} (h) | Tₘₐₓ (h) | Cₘₐₓ (ug/mL) | AUCₗₐₛₜ (h*ug/mL) | AUCINF obs (h*ug/mL) | Cl_obs (mL/h/kg) | MRTₗₐₛₜ (h) | Vss_obs (mL(kg) |
|---|---|---|---|---|---|---|---|---|
| M1 | 210 | 0.333 | 633.2 | 83150 | 86363.2 | 0.23 | 239 | 64.6 |
| M2 | 244 | 0.333 | 474.1 | 101740 | 107088.4 | 0.19 | 280 | 62.4 |
| Average | 227 | 0.333 | 553.7 | 92445 | 96726 | 0.21 | 260 | 63.5 |

## Claims

1. An anti-human PVRIG antibody or an antigen-binding fragment thereof, comprising:
(1) HCDR1 comprising the amino acid sequence of SEQ ID NO: 1, HCDR2 comprising the amino acid sequence of SEQ ID NO: 2, and HCDR3 comprising the amino acid sequence of SEQ ID NO: 3; and/or LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6; or
(2) HCDR1 comprising the amino acid sequence of SEQ ID NO: 17, HCDR2 comprising the amino acid sequence of SEQ ID NO: 18 or SEQ ID NO: 19 or SEQ ID NO: 20, and HCDR3 comprising the amino acid sequence of SEQ ID NO: 21; and/or LCDR1 comprising the amino acid sequence of SEQ ID NO: 22 or SEQ ID NO: 23, LCDR2 comprising the amino acid sequence of SEQ ID NO: 24 or SEQ ID NO: 25, and LCDR3 comprising the amino acid sequence of SEQ ID NO: 26; or
(3) HCDR1 comprising the amino acid sequence of SEQ ID NO: 36, HCDR2 comprising the amino acid sequence of SEQ ID NO: 37, and HCDR3 comprising the amino acid sequence of SEQ ID NO: 38; and/or LCDR1 comprising the amino acid sequence of SEQ ID NO: 39, LCDR2 comprising the amino acid sequence of SEQ ID NO: 40, and LCDR3 comprising the amino acid sequence of SEQ ID NO: 41.

2. The anti-human PVRIG antibody or the antigen-binding fragment thereof according to claim 1, comprising:
(1) HCDR1 comprising the amino acid sequence of SEQ ID NO: 1, HCDR2 comprising the amino acid sequence of SEQ ID NO: 2, and HCDR3 comprising the amino acid sequence of SEQ ID NO: 3; and LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6; or
(2) HCDR1 comprising the amino acid sequence of SEQ ID NO: 17, HCDR2 comprising the amino acid sequence of SEQ ID NO: 20, and HCDR3 comprising the amino acid sequence of SEQ ID NO: 21; and LCDR1 comprising the amino acid sequence of SEQ ID NO: 23, LCDR2 comprising the amino acid sequence of SEQ ID NO: 25, and LCDR3 comprising the amino acid sequence of SEQ ID NO: 26; or
(3) HCDR1 comprising the amino acid sequence of SEQ ID NO: 36, HCDR2 comprising the amino acid sequence of SEQ ID NO: 37, and HCDR3 comprising the amino acid sequence of SEQ ID NO: 38; and LCDR1 comprising the amino acid sequence of SEQ ID NO: 39, LCDR2 comprising the amino acid sequence of SEQ ID NO: 40, and LCDR3 comprising the amino acid sequence of SEQ ID NO: 41.

3. The anti-human PVRIG antibody or the antigen-binding fragment thereof according to any one of claims 1-2, comprising a heavy chain variable region and/or a light chain variable region sequence, wherein:
(1) the heavy chain variable region comprises an amino acid sequence selected from SEQ ID NO: 7, SEQ ID NO: 9, SEQ ID NO: 10, SEQ ID NO: 11 or SEQ ID NO: 12, and/or the light chain variable region comprises an amino acid sequence selected from SEQ ID NO: 8, SEQ ID NO: 13, SEQ ID NO: 14, SEQ ID NO: 15 or SEQ ID NO: 16; or
(2) the heavy chain variable region comprises an amino acid sequence selected from SEQ ID NO: 27, SEQ ID NO: 29, SEQ ID NO: 30, SEQ ID NO: 31 or SEQ ID NO: 32, and/or the light chain variable region comprises an amino acid sequence selected from SEQ ID NO: 8, SEQ ID NO: 33, SEQ ID NO: 34 or SEQ ID NO: 35; or
(3) the heavy chain variable region comprises an amino acid sequence selected from SEQ ID NO: 42, SEQ ID NO: 44, SEQ ID NO: 45, SEQ ID NO: 46 or SEQ ID NO: 47, and/or the light chain variable region comprises an amino acid sequence selected from SEQ ID NO: 43, SEQ ID NO: 48, SEQ ID NO: 49 or SEQ ID NO: 50.

4. The anti-human PVRIG antibody or the antigen-binding fragment thereof according to claim 3, comprising a heavy chain variable region and/or a light chain variable region sequence, wherein:
(1) the heavy chain variable region comprises the amino acid sequence of SEQ ID NO: 47, and/or the light chain variable region comprises the amino acid sequence of SEQ ID NO: 50; or
(2) the heavy chain variable region comprises the amino acid sequence of SEQ ID NO: 32, and/or the light chain variable region comprises the amino acid sequence of SEQ ID NO: 35; or
(3) the heavy chain variable region comprises the amino acid sequence of SEQ ID NO: 10, and/or the light chain variable region comprises the amino acid sequence of SEQ ID NO: 14.

5. The anti-human PVRIG antibody or the antigen-binding fragment thereof according to claim 4, comprising a heavy chain variable region and a light chain variable region sequence, wherein:
(1) the heavy chain variable region comprises the amino acid sequence of SEQ ID NO: 47, and the light chain variable region comprises the amino acid sequence of SEQ ID NO: 50; or
(2) the heavy chain variable region comprises the amino acid sequence of SEQ ID NO: 32, and the light chain variable region comprises the amino acid sequence of SEQ ID NO: 35; or
(3) the heavy chain variable region comprises the amino acid sequence of SEQ ID NO: 10, and the light chain variable region comprises the amino acid sequence of SEQ ID NO: 14.

6. An anti-human PVRIG antibody or an antigen-binding fragment thereof, comprising a heavy chain variable region and/or a light chain variable region sequence, wherein:
(1) the heavy chain variable region comprises a sequence selected from the amino acid sequences respectively having 80%, 85%, 90%, 95% or 99% homology with SEQ ID NO: 7, SEQ ID NO: 9, SEQ ID NO: 10, SEQ ID NO: 11 or SEQ ID NO: 12, and/or the light chain variable region comprises a sequence selected from the amino acid sequences respectively having 80%, 85%, 90%, 95% or 99% homology with SEQ ID NO: 8, SEQ ID NO: 13, SEQ ID NO: 14, SEQ ID NO: 15 or SEQ ID NO: 16; or
(2) the heavy chain variable region comprises a sequence selected from the amino acid sequences respectively having 80%, 85%, 90%, 95% or 99% homology with SEQ ID NO: 27, SEQ ID NO: 29, SEQ ID NO: 30, SEQ ID NO: 31 or SEQ ID NO: 32, and/or the light chain variable region comprises a sequence selected from the amino acid sequences respectively having 80%, 85%, 90%, 95% or 99% homology with SEQ ID NO: 8, SEQ ID NO: 33, SEQ ID NO: 34 or SEQ ID NO: 35; or
(3) the heavy chain variable region comprises a sequence selected from the amino acid sequences respectively having 80%, 85%, 90%, 95% or 99% homology with SEQ ID NO: 42, SEQ ID NO: 44, SEQ ID NO: 45, SEQ ID NO: 46 or SEQ ID NO: 47, and/or the light chain variable region comprises a sequence selected from the amino acid sequences respectively having 80%, 85%, 90%, 95% or 99% homology with SEQ ID NO: 43, SEQ ID NO: 48, SEQ ID NO: 49 or SEQ ID NO: 50.

7. The anti-human PVRIG antibody or the antigen-binding fragment thereof according to claim 6, comprising a heavy chain variable region and/or a light chain variable region sequence, wherein:
(1) the heavy chain variable region comprises a sequence selected from the amino acid sequences respectively having 80%, 85%, 90%, 95% or 99% homology with the amino acid sequence of SEQ ID NO: 47, and/or the light chain variable region comprises a sequence selected from the amino acid sequences respectively having 80%, 85%, 90%, 95% or 99% homology with the amino acid sequence of SEQ ID NO: 50; or
(2) the heavy chain variable region comprises a sequence selected from the amino acid sequences respectively having 80%, 85%, 90%, 95% or 99% homology with the amino acid sequence of SEQ ID NO: 32, and/or the light chain variable region comprises a sequence selected from the amino acid sequences respectively having 80%, 85%, 90%, 95% or 99% homology with the amino acid sequence of SEQ ID NO: 35; or
(3) the heavy chain variable region comprises a sequence selected from the amino acid sequences respectively having 80%, 85%, 90%, 95% or 99% homology with the amino acid sequence of SEQ ID NO: 10, and/or the light chain variable region comprises a sequence selected from the amino acid sequences respectively having 80%, 85%, 90%, 95% or 99% homology with the amino acid sequence of SEQ ID NO: 14.

8. The anti-human PVRIG antibody or the antigen-binding fragment thereof according to any one of claims 1-7, which is a humanized antibody or a human antibody, or a portion thereof.

9. The anti-human PVRIG antibody or the antigen-binding fragment thereof according to any one of claims 1-8, which is an IgG1 or an IgG4 antibody, or a portion thereof.

10. The antigen-binding fragment according to any one of claims 1-9, which is selected from Fab, Fab', F(ab')₂, Fd, Fv, scFv, dAb or CDR.

11. The antigen-binding fragment of any one of claims 1-10, wherein a mutation is present in the Fc region of the antibody; preferably the Fc mutation comprises at least one mutation selected from S239D, A330L and I332E; further preferably the Fc region mutation comprises the S239D, A330L and I332E triple mutations.

12. An isolated nucleic acid molecule, encoding the anti-human PVRIG antibody or the antigen-binding fragment thereof according to any one of claims 1-11.

13. An expression vector, comprising the nucleic acid molecule according to claim 10.

14. A host cell, comprising the expression vector according to claim 13.

15. A method for preparing the anti-human PVRIG antibody or the antigen-binding fragment thereof according to any one of claims 1-11, comprising culturing the host cell according to claim 14, thereby expressing the nucleic acid molecule.

16. The method according to claim 15, further comprising the step of recovering the anti-human PVRIG antibody or the antigen-binding fragment thereof from the host cell culture.

17. An antibody-drug conjugate, comprising the anti-human PVRIG antibody or the antigen-binding fragment thereof according to any one of claims 1-11 linked to a therapeutic agent.

18. A bispecific molecule, comprising the anti-PVRIG antibody or the antigen-binding fragment thereof according to any one of claims 1-11 linked to a peptide targeting another antigenic epitope or another antigen.

19. A composition, comprising: the anti-human PVRIG antibody or the antigen-binding fragment thereof according to any one of claims 1-11, or the antibody-drug conjugate according to claim 17, or the bispecific molecule according to claim 18.

20. Use of the anti-human PVRIG antibody or the antigen-binding fragment thereof according to any one of claims 1-11, or the antibody-drug conjugate according to claim 17, or the bispecific molecule according to claim 18, or the composition according to claim 19 in the preparation of a medicament for regulating immune response in a subject.

21. Use of the anti-human PVRIG antibody or the antigen-binding fragment thereof according to any one of claims 1-11, or the antibody-drug conjugate according to claim 17, or the bispecific molecule according to claim 18, or the composition according to claim 19 in the preparation of a medicament for blocking the binding of PVRIG protein to CD112 protein.

22. Use of the anti-human PVRIG antibody or the antigen-binding fragment thereof according to any one of claims 1-11, or the antibody-drug conjugate according to claim 17, or the bispecific molecule according to claim 18, or the composition according to claim 19 in the preparation of a medicament for inhibiting the growth of tumor cells in a subject.

23. The use according to claim 22, wherein the tumor cells are tumor cells selected from : bone cancer, pancreatic cancer, skin cancer, head or neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, cancer of the endocrine system, thyroid cancer, parathyroid cancer, adrenal cancer, soft tissue sarcoma, urethral cancer, penile cancer, chronic or acute leukemia, acute myeloid leukemia, chronic myeloid leukemia, acute lymphocytic leukemia, chronic lymphocytic leukemia, childhood solid tumor, lymphocytic lymphoma, bladder cancer, kidney or ureter cancer, renal pelvis cancer, central nervous system (CNS) neoplasms, primary CNS lymphomas, tumor angiogenesis, spinal cord axis tumor, brain stem gliomas, pituitary adenomas, Kaposi's sarcoma, epidermoid carcinomas, squamous cell carcinomas, T-cell lymphomas, and any combination of the above cancers.

24. Use of a therapeutically effective amount of the anti-human PVRIG antibody or the antigen-binding fragment thereof accordin g to any one of claims 1-11, or the antibody-drug conjugate according to claim 17, or the bispecific molecule according to claim 18, or the composition according to claim 19 in the preparation of a medicament for treating a hyperproliferative disease.

25. The use according to claim 24, wherein the hyperproliferative disease is a cancer; preferably, the cancer is selected from: bone cancer, pancreatic cancer, skin cancer, head or neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, cancer of the endocrine system, thyroid cancer, parathyroid cancer, adrenal cancer, soft tissue sarcoma, urethral cancer, penile cancer, chronic or acute leukemia, acute myeloid leukemia, chronic myeloid leukemia, acute lymphocytic leukemia, chronic lymphocytic leukemia, childhood solid tumor, lymphocytic lymphoma, bladder cancer, kidney or ureter cancer, renal pelvis cancer, central nervous system (CNS) neoplasms, primary CNS lymphomas, tumor angiogenesis, spinal cord axis tumor, brain stem gliomas, pituitary adenomas, Kaposi's sarcoma, epidermoid carcinomas, squamous cell carcinomas, T-cell lymphomas, and any combination of the above cancers.

26. The use according to any one of claims 20-25, wherein the anti-human PVRIG antibody or the antigen-binding fragment thereof is combined with an anti-PD-1 antibody or an antigen-binding fragment thereof.
